# EUROPEAN PATENT SPECIFICATION

(11) **EP 3 554 501 B1**
(45) Date of publication and mention of the grant of the patent: **09.06.2021**
(21) Application number: 17828942.7
(22) Date of filing: 12.12.2017
(51) Int. Cl.: A61K 31/4174, A61P 25/22, A61K 9/00, A61K 47/20, A61K 9/06, A61K 47/38

(54) **DEXMEDETOMIDINE OR MEDETOMIDINE FOR USE IN TREATING SEPARATION ANXIETY IN COMPANION ANIMALS**
DEXMEDETOMIDIN ODER MEDETOMIDIN ZUR VERWENDUNG BEI DER BEHANDLUNG VON TRENNUNGSANGST BEI BEGLEITTIEREN
DEXMÉDÉTOMIDINE OU MÉDÉTOMIDINE DESTINÉE À ÊTRE UTILISÉE DANS LE TRAITEMENT DE L'ANXIÉTÉ DE SÉPARATION CHEZ LES ANIMAUX DE COMPAGNIE

(30) Priority: 13.12.2016 FI 20165960
(43) Date of publication of application: 23.10.2019
(73) Proprietor: Orion Corporation, 02200 Espoo (FI)
(72) Inventor: KORPIVAARA, Mira, 02101 Espoo (FI); NIINISTÖ, Sauli, 20500 Turku (FI); ORMIO, Sanna, 02100 Espoo (FI); SARÉN, Nina, 02130 Espoo (FI)
(74) Representative: J A Kemp LLP
(86) International application number: PCT/FI2017/050880
(87) International publication number: WO 2018/109272

(56) References cited:
- WO-A1-2014/060638
- NIWAKO OGATA ET AL: "The use of clonidine in the treatment of fear-based behavior problems in dogs: An open trial", JOURNAL OF VETERINARY BEHAVIOR: CLINICAL APPLICATIONS AND RESEARCH, vol. 6, no. 2, 2011, pages 130-137, XP028154405, ISSN: 1558-7878, DOI: 10.1016/J.JVEB.2010.10.004 [retrieved on 2010-10-19]
- Anonymous: "Zoetis Announces Launch of SILEO for Treatment of Noise Aversion in Dogs", Press Releases, 16 May 2016 (2016-05-16), pages 1-5, XP055453245, Retrieved from the Internet: URL:http://news.zoetis.com/press-release/c ompanion-animals/zoetis-announces-launch-s ileo-treatment-noise-aversion-dogs [retrieved on 2018-02-22]
- HARRIS J C ET AL: "Mediation of separation distress by @a"2-adrenergic mechanisms in a non-human primate", BRAIN RESEARCH, ELSEVIER, AMSTERDAM, NL, vol. 410, no. 2, 5 May 1987 (1987-05-05), pages 353-356, XP024267947, ISSN: 0006-8993, DOI: 10.1016/0006-8993(87)90337-4 [retrieved on 1987-05-05]
- PASIN LAURA ET AL: "Dexmedetomidine vs midazolam as preanesthetic medication in children: a meta-analysis of randomized controlled trials.", PAEDIATRIC ANAESTHESIA MAY 2015, vol. 25, no. 5, May 2015 (2015-05), pages 468-476, XP002778509, ISSN: 1460-9592
- Anne E. Cohen ET AL: "Oral transmucosal administration of dexmedetomidine for sedation in 4 dogs", Can Vet, 1 January 2015 (2015-01-01), pages 1144-1148, XP055453254, DOI: 10.1016/S1624-5687(06)71155-6 Retrieved from the Internet: URL:https://www.ncbi.nlm.nih.gov/pmc/artic les/PMC4608466/pdf/cvj_11_1144.pdf [retrieved on 2018-02-22]
- Anonymous: "SILEO (dexmedetomidine oromucosal gel)", DailyMed, 1 February 2016 (2016-02-01), pages 1-6, XP55453269, Retrieved from the Internet: URL:https://dailymed.nlm.nih.gov/dailymed/ fda/fdaDrugXsl.cfm?setid=8113cc85-cd1e-4ed c-b933-51bdbd1d8b64&type=display [retrieved on 2018-02-22]

## Description

### Technical field

The present invention relates to a field of veterinary medicine. In particular, the invention relates to the treatment of separation anxiety in companion animals, particularly dogs. The treatment comprises administering dexmedetomidine or medetomidine or a pharmaceutically acceptable salt thereof to a subject in need of such treatment.

### Background of the invention

Separation anxiety is a behavioral syndrome of companion animals, particularly dogs, characterized by signs of distress when the animal is left alone or is separated from the person or people to whom it is attached. Symptoms include panting, drooling, barking, vocalization, pacing, trembling, urinating, defecating, destructiveness and escape behavior. Typically, the dog's behavior when alone is in marked contrast to its behavior in the presence of the owner, when it may never exhibit anxiety-related behaviors. Some dogs with separation anxiety may also exhibit hyper-attachment such as following the owner around house and staying in close promixity to and touching the owner. Many dogs with separation anxiety appear restless or clingy as the owner initiates his/her departure ritual.

Management of separation anxiety in dogs includes behavior training and medication. Clomipramine (Clomicalm) and fluoxetine (Reconcile) are most often used in the treatment of separation anxiety. Ogata et al. disclose in J. Vet. Behav., 6(2), 2011, 130-137 that the alpha-2 agonist clonidine is useful in the treatment of fear-based problems in dogs, including separation anxiety and noise phobia. WO-A-2014/060638 discloses an oromucosal gel containing dexmedetomidine or medetomidine for use in alleviating noise aversion in companion animals, preferably dogs. Medical therapies authorized for the treatment of separation anxiety in dogs generally involve a long period of onset (several weeks) and, to be effective, must be accompanied by well-designed behavioral training instructions targeted to pet owners. Due to time constraints and lack of knowledge many animal owners are not able to follow these instructions and many dogs remain untreated. Moreover, the current therapy requires a constant medication for several months and as the therapy affects the mood and character of the dog also when the owner is at home, many dog owners have difficulties in committing to the therapy.

Thus, there is a need for an effective medical non-sedative treatment of separation anxiety in companion animals, particularly dogs, having rapid onset of action and sufficiently easy administration such that it could be performed by the pet owner.

Dexmedetomidine and its racemic form medetomidine are alpha-2 adrenoceptor which are commercially available as hydrochloride salt for veterinary sedation in doses which are 375 µg/m² intravenously or 500 µg/m² intramuscularly of dexmedetomidine hydrochloride, and 750 µg/m² intravenously or 1000 µg/m² intramuscularly of medetomidine hydrochloride. Dexmedetomidine hydrochloride is also available for the treatment of noise aversion in dogs as oromucosal gel in doses of 125 µg/m².

### Summary of the invention

It has now been unexpectedly found that separation anxiety in companion animals, particularly dogs, can be effectively alleviated by administering dexmedetomidine or medetomidine or a pharmaceutically acceptable salt thereof in doses that do not produce clinical sedation in subject animals. The separation anxiety alleviating effect was also found to be rapid such that the drawback of long period of onset time associated with current medications is avoided. The therapy of the present invention was also found to be effective even in the absence of behavioral training which is necessary with the currently authorized medications in dogs. It was also found that a single dose of medication according to the present invention was effective to alleviate symptoms of separation anxiety for at least 8 hours, i.e. far longer than the medication appears in the plasma of the animal. Therefore, a constant drug therapy associated with current medications is also avoided.

Previous medication strategies with long-term medication relay on the constant effect of the given medications to the brain neurochemistry. Such changes in neurochemistry then allows the effective use of behaviour modification training with a goal to alleviate the separation anxiety syndrome in general. In contrast, the present invention relies on a totally different strategy where the medication is used only when needed to reduce dog's arousal related to owner's departure rituals and shortly thereafter. Avoiding arousal during this time of most intense reactions by blunting such reactions has been found to have a beneficial effect on the separation tolerance of individual dogs suffering from separation anxiety.

The invention is defined by the claims. Thus, according to one embodiment of the invention, the present invention provides a compound which is dexmedetomidine, medetomidine or a pharmaceutically acceptable salt thereof as the sole active ingredient for use in treating separation anxiety in companion animals, particularly dogs.

According to an aspect of the disclosure, the present disclosure provides a method for treating separation anxiety in companion animals, particularly dogs, comprising administering to a subject in need thereof an effective amount of dexmedetomidine, medetomidine or a pharmaceutically acceptable salt thereof, without producing clinical sedation.

According to another aspect of the disclosure, the present disclosure provides a method for treating separation anxiety in companion animals, particularly dogs, comprising administering to a subject in need thereof an effective amount of dexmedetomidine, medetomidine or a pharmaceutically acceptable salt thereof, wherein the subject retains its ability to stand up and walk without signs of ataxia.

According to another aspect of the disclosure, the present disclosure provides a method for treating separation anxiety in companion animals, particularly dogs, comprising administering to a subject in need thereof an effective amount of dexmedetomidine, medetomidine or a pharmaceutically acceptable salt thereof, wherein the treated animal remains alert and fully functional such that the animal's ability to eat, move or respond to stimuli is not impaired.

According to another embodiment of the invention, the present invention provides an oromucosal gel formulation comprising, per weight of the composition, 0.001 to about 0.2 % (w/w) of dexmedetomidine, medetomidine or a pharmaceutically acceptable salt thereof, 0.3-40% (w/w) of a gelling agent; 0.2-15% (w/w) of a transmucosal penetration enhancer; 5-50% (w/w) of a water-miscible organic co-solvent; and 30-80% (w/w) of water, for use in treating separation anxiety in companion animals, particulary dogs, wherein dexmedetomidine, medetomidine or a pharmaceutically acceptable salt thereof is administered as the sole active ingredient, wherein a single dose of the gel formulation is administered and where the does is effective to alleviate symtoms of separation anxiety within 60 minutes, preferably within 45 minutes, most preferably within 30 minutes. According to one aspect, the effect lasts for at least 8 hours.

According to another aspect of the disclosure, the present disclosure provides a method for treating separation anxiety in companion animals, particularly dogs, comprising applying effective amount of a composition in a form a transmucosal gel comprising dexmedetomidine, medetomidine or a pharmaceutically acceptable salt thereof as an active ingredient, on the mucosa, particularly oral mucosa, of a companion animal, particularly a dog.

In still further aspect and according to any of the above aspects of the disclosure, the present disclosure provides a method for treating separation anxiety in companion animals, particularly dogs, comprising administering to a subject in need thereof an effective amount of dexmedetomidine, medetomidine or a pharmaceutically acceptable salt thereof, wherein the treatment is continued until the symptoms of separation anxiety gradually disappear or are reduced over time such that the animal no longer requires the treatment.

According to one aspect of the disclosure, the present disclosure provides a veterinary kit comprising a) a composition in the form of a transmucosal gel comprising dexmedetomidine, medetomidine or a pharmaceutically acceptable salt thereof as an active ingredient, b) a package for containing said composition, and c) instructions for administering said composition on the mucosa, particularly oral mucosa, of an animal, particularly a dog, for alleviating separation anxiety.

According to one embodiment of the invention, dexmedetomidine, or a pharmaceutically acceptable salt thereof, particularly hydrochloride salt, is used as an active ingredient. According to another embodiment of the invention, medetomidine or a pharmaceutically acceptable salt thereof, particularly hydrochloride salt, is used as an active ingredient.

### Detailed description of the invention

The term "separation anxiety", as used herein, refers to behavioral syndrome of companion animals, particularly dogs, characterized by signs of distress when the animal is left alone or is separated from the person or people to whom it is attached. Typical symptoms of separation anxiety include panting, drooling, barking, vocalization, pacing, trembling, urinating, defecating, destructiveness and escape behavior.

The term "clinical sedation", as used herein, means a state of relaxation characterized by reduced vigilance/alertness and depression of central nervous system functions without total loss of consciousness. Animals appear to be immobilized and sleeping (e.g. dogs are lying on the surface) and do not respond to normal stimulus. Clinical sedation in dogs in a study setting can be defined for instance by posture (lying ± rising with difficulty or unable to rise), jaw tone (weakened or very weak), response to noise (no reaction) and ability to perform a particular procedure which requires sedation and restraint.

The term "companion animal", as used herein, refers to an animal suitable for being kept as a pet by humans and includes dog and cat.

The term "alleviating", as used herein, refers to reducing, inhibiting, preventing, suppressing or removing symptoms of separation anxiety.

The present invention relates to a compound which is dexmedetomidine, medetomidine or a pharmaceutically acceptable salt thereof as the sole active ingredient for use in treating separation anxiety in companion animals, particularly dogs. Dexmedetomidine, medetomidine or a pharmaceutically acceptable salt thereof are found to be effective in alleviating separation anxiety in doses which do not produce clinical sedation in subject animals. Thus, the treated animals remain alert and fully functional such that the treatment does not impair the animal's ability to eat, move or respond to stimuli (e.g. owner calling the dog).

Dexmedetomidine, medetomidine or a pharmaceutically acceptable salt thereof can be administered to a subject animal suffering from separation anxiety e.g. by intravenous or intramuscular route. However, preferably the active ingredient of the invention is administered to a subject animal transmucosally, preferably to oral mucosa of the animal (oromucosally). The active ingredient can be delivered oromucosally using compositions well known in the art, such as patches, wafers, films, solutions or semisolid compositions such as emulsions or gels. In particular, it is preferred to administer dexmedetomidine, medetomidine or a pharmaceutically acceptable salt thereof to a subject animal in the form of a semisolid composition such as an oromucosal gel.

The amount of the active ingredient to be administered is suitably selected such as to provide sufficient separation anxiety alleviating effect without undesired signs of clinical sedation. Accordingly, for alleviating separation anxiety in companion animals such as dog, dexmedetomidine or a pharmaceutically acceptable salt thereof, preferably hydrochloride salt, is suitably administered in an amount that produces plasma Cₘₐₓ concentration of dexmedetomidine which is from about 0.05 to about 0.8 ng/ml, more typically from about 0.1 to about 0.7 ng/ml, preferably from about 0.15 to about 0.6 ng/ml, more preferably from about 0.2 to about 0.5 ng/ml, for example from about 0.3 to about 0.4 ng/ml. Medetomidine or a pharmaceutically acceptable salt thereof, preferably hydrochloride salt, is suitably administered in an amount that produces plasma Cₘₐₓ concentration of medetomidine which is from about 0.1 to about 1.4 ng/ml, preferably from about 0.3 to about 1.2 ng/ml, more preferably from about 0.4 to about 1.0 ng/ml, for example from about 0.5 to about 0.8 ng/ml.

The actual amount of the drug to be administered may depend on numerous factors, such as the species, age and weight of the subject to be treated, the active ingredient used, route of administration and the type of the composition.

For treating separation anxiety in dog using oromucosal administration, dexmedetomidine or a pharmaceutically acceptable salt thereof, preferably hydrochloride salt, is administered suitably in an amount of about 10 µg/m² to about 200 µg/m², preferably from about 20 µg/m² to about 180 µg/m², more preferably from about 30 µg/m² to about 150 µg/m², wherein the unit µg/m² refers to micrograms of active agent per square metre body surface area of the subject animal. For treating separation anxiety in dog using oromucosal administration, medetomidine or a pharmaceutically acceptable salt thereof, preferably hydrochloride salt, is administered suitably in an amount of about 20 µg/m² to about 400 µg/m², preferably from about 40 µg/m² to about 360 µg/m², more preferably from about 60 µg/m² to about 300 µg/m², wherein the unit µg/m² is as explained above. Using the oromucosal semisolid gel according to the present invention, dexmedetomidine or a pharmaceutically acceptable salt thereof, preferably hydrochloride salt, is administered preferably in an amount of 50 to 200 µg/m², preferably from 70 µg/m² to 180 µg/m², more preferably from 100 µg/m² to 150 µg/m², and medetomidine or a pharmaceutically acceptable salt thereof, preferably hydrochloride salt, in an amount of 100 to 400 µg/m², preferably from 140 µg/m² to 360 µg/m², more preferably from 200 µg/m² to 300 µg/m².

For treating separation anxiety in dog using intramuscular injection, dexmedetomidine or a pharmaceutically acceptable salt thereof, preferably hydrochloride salt, is administered generally in an amount of about 1 µg/m² to about 40 µg/m², preferably from about 5 µg/m² to about 30 µg/m², for example from about 10 µg/m² to about 20 µg/m², wherein the unit µg/m² is as explained above. For treating separation anxiety in dog using intramuscular injection, medetomidine or a pharmaceutically acceptable salt thereof, preferably hydrochloride salt, is administered suitably in an amount of about 2 µg/m² to about 80 µg/m², preferably from about 10 µg/m² to about 60 µg/m², for example from about 20 µg/m² to about 40 µg/m², wherein the unit µg/m² is as explained above.

Weight to body surface area conversion charts for dogs are readily available in veterinary handbooks which are well known to a person skilled in the art.

The semisolid composition useful in method of the invention may be for example a gel, cream, ointment or paste. Preferred composition is in the form of a gel or emulsion. Gel form is particularly preferred.

Semisolid dosage forms for use according to the invention can be prepared by methods well known in the art. They can be prepared by combining the drug substance with conventional pharmaceutical diluents and carriers commonly used in semisolid formulations.

The particularly suitable semisolid pharmaceutical veterinary composition for use in the present invention is a semisolid gel form adapted for transmucosal administration comprising dexmedetomidine or medetomidine or a pharmaceutically acceptable salt thereof as an active ingredient. The term "semisolid" mean here the mechano-physical state that is flowable under moderate stress. Preferably, the composition is easily syringable, meaning that it can readily be dispensed from a conventional tube of the kind well known for topical formulations or from needleless syringe. The semisolid composition should be viscous enough for being able to remain in the mouth of the animal, however the viscosity should not be so high that the composition could be easily swallowed. Preferably, the semisolid material should have a viscosity from about 500 to about 200,000 mPas, preferably from about 1,000 to about 100,000 mPas, more preferably from about 5,000 to about 50,000 mPas, for example from about 8,000 to about 30,000 mPas. According to one embodiment, the semisolid material has a viscosity from about 3000 mPas to about 50,000 mPas, particularly from about 5,000 mPas to about 20,000 mPas.

The semisolid gel of the present invention has a spreadable consistency upon administration and has been found to be non-irritating even after multiple administrations. Thus, the present composition differs from transmucosal compositions which are in the form of a patch, matrix, film or wafer, which dosage forms may have a drawback of potential irritation of the mucosa.

The gel composition can be applied on any suitable mucosa of an animal including oral, nasal, vaginal and rectal mucosa. In particular, the composition is suitably applied on the oral mucosa of an animal, e.g. buccal, lingual, sublingual or gingival mucosa. For a dog, it is preferably applied to the buccal and/or gingival mucosa, from where the active ingredient is absorbed through the mucous membranes of the oral cavity into the circulation and induces the desired pharmacological effect. The gel composition is suitably applied oromucosally in a small volume using a suitable applicator such as a syringe or the like. The composition remains in its application place and is not readily swallowed. The administration of the semisolid dosage is easy and can be performed by the animal owner or handler who is not skilled in parenteral drug administration. The onset of the separation anxiety alleviating effect is rapid, and generally starts in dog within 60 minutes, preferably within 45 minutes, most preferably within 30 minutes, from the time of application. The separation anxiety alleviating effect of a single dose of the oromucosal gel composition lasts typically at least 8 hours, even though the medication appears typically only 3-4 hours in the plasma of the animal.

Gel, as referred to herein, is a single phase semisolid system consisting of organic macromolecules (gelling agent) uniformly distributed throughout a liquid in such a manner that no apparent boundaries exists between the dispersed macromolecules and the liquid. A veterinary transmucosal composition in the form of a gel has been found to be a particularly suitable for use in the invention.

Gel structure is obtainable by using a suitable gelling agent. The amount of gelling agent is selected such that the resulting gel has the desired rheological properties. The gel according to the invention is preferably an aqueous gel (hydrogel), wherein the liquid solvent comprises water. However, the aqueous gel formulation may also comprise suitable water-miscible co-solvents. The active ingredient is uniformly dissolved or dispersed in the gel composition.

Preferably, the transmucosal gel formulation for use according to the invention comprises dexmedetomidine, medetomidine or a pharmaceutically acceptable salt thereof, a gelling agent, a transmucosal penetration enhancer, water-miscible organic co-solvent and water.

The concentration of dexmedetomidine, medetomidine or a pharmaceutically acceptable salt thereof in the oromucosal composition, e.g. in the semisolid gel composition, is suitably within the range of about 0.001 to about 0.2 % (w/w), preferably from about 0.002 to about 0.1 % (w/w), suitably from about 0.005 to about 0.05 % (w/w), per weight of the composition.

Pharmaceutically acceptable salts of dexmedetomidine and medetomidine can be prepared by known methods. Suitable salts include acid addition salts formed, for example, with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid and the like. Hydrochloride is the preferred salt.

The gelling agent may be any suitable hydrophilic gel forming polymer. Preferably, the gelling agent is selected from cellulose derivatives, polyacrylic acids and polyoxyethylene/polyoxypropylene copolymers. Cellulose derivatives and polyacrylic acids are particularly preferred gelling agents.

Suitable cellulose derivatives for use as gelling agents include cellulose ethers such as hydroxypropyl cellulose, hydroxyethyl cellulose, hydroxyethyl methylcellulose, methylcellulose, hydroxypropyl methylcellulose, hydroxypropyl ethylcellulose, hydroxycellulose and the like. Preferred cellulose ethers include hydroxypropyl cellulose and hydroxyethyl cellulose.

Suitable polyacrylic acids for use as gelling agents include carbomers (also called carboxyvinyl polymers). Carbomers are polyalkenyl polyether cross-linked polymers acrylic acids, typically polyallyl sucrose or polyallyl pentaerythritol cross-linked polymers of acrylic acid. They are available e.g. under the trade name Carbopol in various grades. Aqueous carbomer dispersions are acidic due to free carboxyl groups of the carbomer polymer. Neutralization of the aqueous dispersions of carbomer polymers causes spontaneous thickening through formation of water-soluble salts of polymer resins.

The gel should be viscous enough for being able to remain in the mouth of the animal, however the viscosity should not be so high that the gel could be easily swallowed by the animal.

The gelling agents are generally used in an amount suitable to provide a gel with a viscosity from about 500 to about 200,000 mPas, preferably from about 1,000 to about 100,000 mPas, more preferably from about 5,000 to about 50,000 mPas, for example from about 8,000 to about 30,000 mPas, measured on a Brookfield Digital Viscometer DV-II, LV-4 (cylindrical spindle), spindle factor 64, 12 rpm, 25°C. According to one embodiment, gelling agents are used in an amount suitable to provide a gel with a viscosity from about 3000 mPas to about 50,000 mPas, particularly from about 5,000 mPas to about 20,000 mPas.

Such suitable viscosity may be obtained by adjusting the amount of gelling agent and/or by adjusting the pH of the composition. This is especially relevant where the gelling agent is a polyacrylic acid such as carbomer as its viscosity is dependent on the pH of the composition.

The amount of the gelling agent depends on the nature of the gelling agent and the desired viscosity. It is preferred that the gel has a spreadable consistency which allows easy oromucosal administration of a small volume of the gel from a syringe or the like. Preferably, the gel composition of the invention is free of bioadhesive components, such as elastomers or the like. Moreover, the gel composition of the invention is preferably not a film-forming type gel composition.

Generally the amount of the gelling agent in the composition for use according to the invention is from about 0.3 to about 40 % (w/w), per weight of the composition. In case where the gelling agent is a cellulose derivative, the amount of the gelling agent is typically from about 0.5 to about 40 % (w/w), more preferably from about 1 to about 30 % (w/w), per weight of the composition. In case where the gelling agent is a polyacrylic acid such as carbomer, the amount of the gelling agent is typically from about 0.3 to about 5.0 % (w/w), more preferably from about 0.5 to about 3.0 % (w/w), per weight of the composition.

In case where the gelling agent is hydroxypropyl cellulose, it is suitably used in an amount ranging from about 5 to about 40 % (w/w), preferably from about 10 to about 25 % (w/w), per weight of the composition.

The pH of the composition is suitably within the range of from about 3 to about 9, preferably from about 4 to about 8, more preferably from about 4.5 to about 7, more preferably from about 5 to about 7, more preferably from about 5.5 to about 6.5, particularly between about 5.8 and 6.2. According to one embodiment, the pH of the composition is within the range of from about 5 to about 6.5. The pH may be adjusted with a suitable basic compound, such as sodium hydroxide, fatty amine or a tertiary amine, or with an acidic compound, such as hydrochloric acid. A gelling agent is typically a slightly acidic material.

Transmucosal penetration enhancers are agents capable of increasing the rate at which the drug permeates through the mucosal membranes and enters the bloodstream. Suitable transmucosal penetration enhancers include for example surfactants, e.g. anionic surfactants such as salts of fatty acids of 5 to 30 carbon atoms, e.g. sodium lauryl sulphate and other sulphate salts of fatty acids, cationic surfactants such as alkylamines of 8 to 22 carbon atoms, e.g. oleylamine, and nonionic surfactants such as polysorbates and poloxamers; aliphatic monohydric alcohols of 8 to 22 carbon atoms such as decanol, lauryl alcohol, myristyl alcohol, palmityl alcohol, linolenyl alcohol and oleyl alcohol; fatty acids of 5 to 30 carbon atoms such as oleic acid, stearic acid, linoleic acid, palmitic acid, myristic acid, lauric acid and capric acid and their esters such as ethyl caprylate, isopropyl myristate, methyl laurate, hexamethylene palmitate, glyceryl monolaurate, polypropylene glycol monolaurate and polyethylene glycol monolaurate; diethyleneglycol monoethyl ether (Transcutol); menthol and other essential oils; salicylic acid and its derivatives; alkyl methyl sulfoxides such as decyl methyl sulfoxide and dimethyl sulfoxide (DMSO); 1-substituted azacycloalkan-2-ones such as 1-dodecylazacyclo-heptan-2-one sold under the trademark AZONE; amides such as octylamide, oleicamide, hexamethylene lauramide, lauric diethanolamide, polyethylene glycol 3-lauramide, N,N-diethyl-m-toluamide and crotamiton; and any other compounds compatible with dexmedetomidine or medetomidine and having transmucosal permeation enhancing activity. One or several of the above transmucosal penetration enhancers can be used. The amount of the transmucosal penetration enhancer in the composition is generally from about 0.1 to about 20 % (w/w), preferably from about 0.2 to about 15 % (w/w), more preferably from about 0.5 to about 10 % (w/w) per weight of the composition, depending on the transmucosal permeation enhancer used.

Preferred transmucosal penetration enhancers are fatty acids of 5 to 30 carbon atoms, particularly isopropyl myristate; sulphate salts of 5 to 30 carbon fatty acids, particularly sodium lauryl sulphate; and DMSO. Sodium lauryl sulphate is particularly preferred.

In case the transmucosal penetration enhancer is sodium lauryl sulphate, it is used in an amount ranging from about 0.1 to about 5 % (w/w), preferably from about 0.2 to about 3 % (w/w), suitably from about 0.5 to about 2 % (w/w), per weight of the composition.

Water-miscible organic co-solvents suitable for use in the gel compositions for use according to the present invention include polyalcohols or glycols such as propylene glycol, butylene glycol, ethylene glycol, preferably propylene glycol or C₂-C₄ alkanols such as ethanol, isopropanol, n-propanol or butanol; or combinations thereof. Preferred are nonvolatile organic co-solvents, particularly propylene glycol. The amount of the water-miscible organic co-solvent in the composition is generally from about 5 to about 50 % (w/w), preferably from about 10 to about 45 % (w/w), more preferably from about 15 to about 40 % (w/w), for example from about 20 to about 35 % (w/w), per weight of the composition.

The amount of water in the gel composition is generally from about 15 to about 90 % (w/w), preferably from about 20 to about 80 % (w/w), more preferably from about 30 to about 75 % (w/w), for example from about 40 to about 70 % (w/w), per weight of the composition.

According to one preferred embodiment, the oromucosal gel formulation comprises per weight of the composition, 0.001 to about 0.2 % (w/w) of dexmedetomidine, medetomidine or a pharmaceutically acceptable salt thereof, 0.3 - 40 % (w/w) of a gelling agent; 0.2 - 15 % (w/w) of a transmucosal penetration enhancer; 5 - 50 % (w/w) of a water-miscible organic co-solvent; and 30 - 80 % (w/w) of water.

According to another preferred embodiment, the oromucosal gel formulation comprises per weight of the composition, 0.005 to about 0.1 % (w/w) of dexmedetomidine, medetomidine or a pharmaceutically acceptable salt thereof, 1 - 30 % (w/w) of a gelling agent; 0.5 - 10 % (w/w) of a transmucosal penetration enhancer; 5 - 50 % (w/w) of a water-miscible organic co-solvent; and 40 - 70 % (w/w) of water.

According to another preferred embodiment, the oromucosal gel formulation comprises, per weight of the composition, 0.005 to about 0.05 % (w/w) of dexmedetomidine, medetomidine or a pharmaceutically acceptable salt thereof, 10 - 25 % (w/w) of hydroxypropyl cellulose; 0.1 - 5 % (w/w) of sodium lauryl sulphate; 15 - 40 % (w/w) of a water-miscible organic co-solvent; and 40 - 70 % (w/w) of water.

The gel composition for use according to the invention can optionally also include other excipients commonly used in the art, for example, preservatives and/or antioxidants such as benzyl alcohol, methyl and propyl parabens, butylhydroxytoluene or butylhydroxyanisole; sweeteners; colouring agents; flavouring agents; buffers; pH adjusting agents; and solubilizers such as glycerol and the like.

The composition for use according to the invention is preferably given to a subject animal oromucosally from a prefilled syringe in a volume ranging from about 0.05 to 5 ml, more preferably from about 0.1 to 2 ml, still more preferably from about 0.2 to 1.5 ml, for example 0.5 ml.

The composition for use according to the invention comprises preferably a colouring agent. For example, a coloured gel can be easily distinguished from saliva following the administration. If the gel product is discharged from the mouth of the animal the owner will be able to note the approximate loss of gel. The owner will also easily note any accidental dosing in case the product comes into contact with his skin.

The composition can be provided in the form of a veterinary kit that comprises the composition for use according to the invention, a package for containing said composition, and instructions for administering said composition on the oral mucosa of a companion animal, particularly dog, for treating separation anxiety. Preferably, said package is an applicator, e.g. a syringe capable of dosing fixed volumes of the composition of the invention. Syringe is preferably prepared form polymer material, such as HDPE. Suitably, the volume of the syringe ranges from about 0.25 to 6 ml, typically from about 0.5 to 5 ml, more typically from about 1 to 5 ml. For example, composition of the invention can be packaged into 1 ml, 2 ml, 4 ml or 5 ml HDPE syringes.

According to one embodiment of the invention, the present invention provides an oromucosal gel formulation for use in a method for treating separation anxiety in companion animals, particularly dogs, comprising administering to a subject in need thereof a single dose of the oromucosal gel formulation comprising, per weight of the composition, 0.001 to about 0.2 % (w/w) of dexmedetomidine, medetomidine or a pharmaceutically acceptable salt thereof, 0.3 - 40 % (w/w) of a gelling agent; 0.2 - 15 % (w/w) of a transmucosal penetration enhancer; 5 - 50 % (w/w) of a water-miscible organic co-solvent; and 30 - 80 % (w/w) of water, wherein the dose is effective to alleviate symptoms of separation anxiety within 60 minutes, preferably within 45 minutes, most preferably within 30 minutes. Preferably the effect lasts for at least 8 hours, for example 8 to 12 hours.

The animal suffering from separation anxiety is suitably treated with the composition for use according to the present invention each time when the symptoms of separation anxiety can be expected, for example daily, or on one or more days of the week. Preferably, the treatment is continued until the symptoms of separation anxiety gradually disappear or are reduced over time such that the animal no longer requires the treatment.

Typically, the animal suffering from separation anxiety is treated with a single dose of the composition for use according to the invention in the morning of each working day about 30 - 60 min prior to the animal is being left alone. The effect of a single dose has been found to be effective for at least 8 hours i.e. longer than the medication appears in the plasma of the animal. Therefore, the animal typically does not show symptoms of separation anxiety when the owner returns home after his/her working day.

The treatment of the present invention can be combined, if desired, with behavioral training that is commonly used in the treatment of separation anxiety of companion animals, particularly dogs. However, the treatment of the present invention has been found to be effective even in the absence of conventional behavioural training.

The invention is further illustrated by the following examples, which are not meant to limit the scope of the invention.

### Example 1. Oromucosal gel of dexmedetomidine HCl

| Ingredient | % (w/w) |
|---|---|
| Dexmedetomidine HCl | 0.01 |
| Hydroxypropyl Cellulose | 15 |
| Propylene Glycol | 30 |
| Sodium Lauryl Sulphate | 1 |
| Sodium Hydroxide (2 M) | q.s. |
| Hydrochloric acid, dilute | q.s. |
| Brilliant Blue FCF (E133) | 0.003 |
| Water | 53.987 |

### Example 2. Oromucosal gel of medetomidine HCl

| Ingredient | % (w/w) |
|---|---|
| Medetomidine HCl | 0.02 |
| Hydroxypropyl Cellulose | 15 |
| Propylene Glycol | 30 |
| Sodium Lauryl Sulphate | 1 |
| Sodium Hydroxide (2 M) | q.s. |
| Hydrochloric acid, dilute | q.s. |
| Brilliant Blue FCF (E133) | 0.003 |
| Water | 53.977 |

The gel formulations of Example 1 and 2 were prepared by adding propylene glycol, colouring agent, sodium lauryl sulphate and water in a vessel. The mixture was stirred until it was miscible and homogenous. The mixture was warmed to 50°C. Hydroxypropyl cellulose was slowly added under stirring. The gel was cooled to room temperature under gentle stirring and drug substance was added under stirring. pH of the composition was adjusted to 6.0 by dropwise addition ofHCl solution. Clear gel was obtained after standing. Gel was packaged into 4 ml HDPE syringes.

### Example 3.

A 10 year old neutered female Cairn terrier started to show symptoms of separation anxiety after a change in her living arrangements. The symptoms included restlessness, panting and following on the heels of the owner and trying to force herself out at the time the owner left the apartment. When the owner returned after the working day, the dog was still shaking and panting behind the door in a freeze position. Oromucosal dexmedetomidine gel was administered to buccal/gingival mucosa of the dog with a syringe using a dose of 125 µg/m² of dexmedetomidine hydrochloride in the morning roughly 30 min prior to the departure of the owner. The panting, following and clinginess had already started 20 min prior to administration. Roughly 15 min after administration the dog started to pant less, was not clinging to the heels of the owner anymore but rather stayed a meter away. After another 5 min the panting was totally gone and the the dog layed peacefully on the floor. After an additional 5 min the dog made a "sigh of relief' and went to her own bed to lay down. The dog was calmly looking from her own bed, like she used to do before development of separation anxiety, at the owner who was getting ready for work. When the owner left the apartment, the dog did not try to follow the owner out, but just laid on the bed looking calmly. When the owner returned home after about 10 hours, the dog woke up from her bed, was stretching and came to greet the owner like she typically did before development of separation anxiety. The medication was then given every working day morning during 3 months (every second week due to living arrangements). The dog actually started to show less symptoms in the mornings before the owner left to work. After 3 months the owner left the medication away and the dog was doing fine without symptoms of separation anxiety.

### Example 4.

A 6 year old female Bichon Frise started to show symptoms of separation anxiety after the owner started to work full time. The symptoms included barking for several hours after the owners left home and frequent vomiting. A dog behaviour specialist diagnosed the dog with separation anxiety and recommended treatment with clomipramine combined with behavioural training. The treatment was not initiated. Instead, oromucosal dexmedetomidine gel was administered to buccal/gingival mucosa of the dog with a syringe using a dose of 125 µg/m² of dexmedetomidine hydrochloride about 30 min prior to the last person left home. The symptoms of separation anxiety eased right away and the owners could leave the dog home without constant barking which earlier could be heard through the front door. The medication was thereafter given each time the dog was left home for longer periods. The medication was effective each time and the constant barking was gone which was also confirmed by the neighbors. The frequent vomiting did also disappear.

### Example 5.

A male mixed breed dog of 9 kg weight started to show symptoms of separation anxiety. The symptoms included barking and whining heavily after about 10 min since the owners left home and lasting the whole day. Neighbours complained about the loud noises. A traditional treatment of separation anxiety was tried first by leaving the dog alone for couple of seconds, then coming back and rewarding the dog for calm behaviour, with gradual prolongation of the separation periods. However, the treatment did not bring any improvement. Oromucosal dexmedetomidine gel was then administered to buccal/gingival mucosa of the dog with a syringe using a dose of 125 µg/m² of dexmedetomidine hydrochloride about 30 min prior to the owner's departure for five consecutive days (Monday to Friday). Within this administration period the dog did not bark. After the first administration period the dog barked every day for the first 3 hours and then stopped. A second administration period was conducted similarly to the first and, again, the dog did not bark during the administration period. After the second administration period the situation was stabilized such that the dog barks only for the first 15 minutes after the owners leave home and is calm afterwards. The dog learned to tolerate significantly better the separation from the owners. Also the neighbours' complainments stopped.

### Example 6.

A male 3 years old mixed breed dog suffering from separation anxiety was treated with conventional therapy including behavioural modification supported by treatment with fluoxetine. The therapy enabled trouble free leaving dog at home during regular daily work departures. However, the dog still showed signs of sepration anxiety to unscheduled departures that were not related to work, especially in the evening. Oromucosal dexmedetomidine gel was then administered to buccal/gingival mucosa of the dog with a syringe using a dose of 125 µg/m² of dexmedetomidine hydrochloride about 15 minutes prior to unscheduled departures such as shopping and visiting friends. After treating the dog during five of these kind of departures the dog started dealing with the unscheduled departures equally well as the regular ones.

### Example 7.

Study protocol for confirming the beneficial effect of dexmedetomidine hydrochloride oromucosal gel in the treatment of separation anxiety in dogs:
Study centres: A multinational study in approximately 10-15 centres.

Objectives: The objectives of this study are to confirm the efficacy and clinical safety of dexmedetomidine hydrochloride oromucosal gel against placebo for the alleviation of symptoms of separation anxiety in dogs suffering from separation anxiety. The product may be given up to 3 times daily as needed 5-7 times a week during the 2-week treatment period.

Study design: Randomised, double-blind, placebo-controlled parallel-group, clinical field study.

Main eligibility criteria: Owner informed consent (IC) obtained from client-owned dogs of any breed or sex, age ≥ 6 months and ≤ 8 years, weight ≥ 2 kg, American Society of Anesthesiologists (ASA)-status I or II. The dog has been with the current owner ≥ 3 months. The dog is house-trained. There are at least five separation anxiety related departures/week. Based on behaviour history and video recordings during the baseline evaluation, the dog has been exhibiting at least 1 of the following signs of separation anxiety related to owner departure: destructive behaviour, restlessness/pacing, vocalization, inappropriate urination or inappropriate defecation.

Number of study animals: Approximately 70 dogs will be enrolled in the study to obtain data of 60 randomised dogs.

Investigational product: Dexmedetomidine hydrochloride 0.1 mg/ml oromucosal gel for dogs at dose of 125 µg/m² administered by the owner as needed up to 3 times a day with a minimum of 2 h intervals on to the oral mucosa between the cheek and gum of the dog for 2 weeks.

Reference product: Placebo oromucosal gel administered by the owner as needed up to 3 times a day with a minimum of 2 h intervals on to the oral mucosa between the cheek and gum of the dog for 2 weeks.

Variables and methods of assessments: Efficacy:
Primary efficacy variable: Assessment of dog's separation anxiety signs related to owner departure. The first medicated departure of the day will be assessed by two independent experts from the video recording for at least 30 minutes according to Cannas, S. et al., J. Vet. Behav.: Clin. Appl. Res. 9, 50-57, 2014, and taking into account further possible signs of dog's behaviour during separation not visible in video recording (destruction or elimination in rooms not visible in the video etc.) as reported by the owner.

Secondary efficacy variable: Owner assessment of the effect of the study treatment on separation anxiety related to owner departures.

Other efficacy variables: Usability of the product.

Safety: Physical examination, laboratory safety variables, observational alertness and functional alertness, and adverse events.

Statistical methods: The primary efficacy variable (duration of separation anxiety signs) will be analysed with a repeated measures analyses of covariance (RM-ANCOVA) model. Secondary and other efficacy variables will be analysed using statistical models and/or descriptive statistics, as appropriate. Safety variables will be analysed using descriptive statistics except differences between treatment groups in the owner alertnessess assessments will be analysed using appropriate statistical model.

## Claims

1. A compound which is dexmedetomidine, medetomidine or a pharmaceutically acceptable salt thereof as the sole active ingredient for use in treating separation anxiety in companion animals, particularly dogs.

2. A compound for use according to claim 1, wherein dexmedetomidine, medetomidine or a pharmaceutically acceptable salt thereof is administered oromucosally.

3. A compound for use according to claim 1 or 2, wherein dexmedetomidine, medetomidine or a pharmaceutically acceptable salt thereof is administered in the form of a semisolid oromucosal gel.

4. A compound for use according to claim 3, wherein the semisolid oromucosal gel comprises, per weight of the composition, 0.001 - 0.2 % (w/w) of dexmedetomidine, medetomidine or a pharmaceutically acceptable salt thereof; 1 - 40 % (w/w) of a gelling agent; 0.2 - 10 % (w/w) of a transmucosal penetration enhancer; 5 - 50 % (w/w) of a water-miscible organic co-solvent; and 30 - 80 % (w/w) of water.

5. A compound for use according to any of claims 1 to 4, wherein the plasma Cₘₐₓ value of dexmedetomidine in the subject animal is from 0.05 to 0.8 ng/ml, preferably from 0.15 to 0.6 ng/ml, more preferably from about 0.2 to about 0.5 ng/ml.

6. A compound for use according to any of claims 1 to 4, wherein the plasma Cₘₐₓ value of medetomidine in the subject animal is from about 0.1 to about 1.4 ng/ml, preferably from about 0.3 to about 1.2 ng/ml, more preferably from about 0.4 to about 1.0 ng/ml.

7. A compound for use according to any of claims 2 to 5, wherein dexmedetomidine or a pharmaceutically acceptable salt thereof is administered oromucosally in an amount of 10 µg/m² to 200 µg/m², preferably from 20 µg/m² to 180 µg/m², more preferably from 30 µg/m² to 150 µg/m².

8. A compound for use according to any of claims 2, 3, 4 or 6, wherein medetomidine or a pharmaceutically acceptable salt thereof is administered oromucosally in an amount of 20 µg/m² to 400 µg/m², preferably from 40 µg/m² to 360 µg/m², more preferably from 60 µg/m² to 300 µg/m².

9. An oromucosal gel formulation comprising, per weight of the composition, 0.001 to about 0.2 % (w/w) of dexmedetomidine, medetomidine or a pharmaceutically acceptable salt thereof, 0.3 - 40 % (w/w) of a gelling agent; 0.2 - 15 % (w/w) of a transmucosal penetration enhancer; 5 - 50 % (w/w) of a water-miscible organic co-solvent; and 30 - 80 % (w/w) of water, for use in treating separation anxiety in companion animals, particularly dogs, wherein dexmedetomidine, medetomidine or a pharmaceutically acceptable salt thereof is administered as the sole active ingredient, wherein a single dose of the gel formulation is administered and wherein the dose is effective to alleviate symptoms of separation anxiety within 60 minutes, preferably within 45 minutes, most preferably within 30 minutes.

10. An oromucosal gel formulation for use according to claim 9, wherein the effect lasts for at least 8 hours.

11. An oromucosal gel formulation for use according to claim 9 or 10, comprising per weight of the composition, 0.005 to about 0.1 % (w/w) of dexmedetomidine, medetomidine or a pharmaceutically acceptable salt thereof, 1 - 30 % (w/w) of a gelling agent; 0.5 - 10 % (w/w) of a transmucosal penetration enhancer; 5 - 50 % (w/w) of a water-miscible organic co-solvent; and 40 - 70 % (w/w) of water.

12. An oromucosal gel formulation for use according to claim 11, comprising, per weight of the composition, 0.005 to about 0.05 % (w/w) of dexmedetomidine, medetomidine or a pharmaceutically acceptable salt thereof, 10 - 25 % (w/w) of hydroxypropyl cellulose; 0.1 - 5 % (w/w) of sodium lauryl sulphate; 15 - 40 % (w/w) of a water-miscible organic co-solvent; and 40 - 70 % (w/w) of water.

13. An oromucosal gel formulation for use according to claim 12, comprising, per weight of the composition, 0.005 to about 0.05 % (w/w) of dexmedetomidine or a pharmaceutically acceptable salt thereof, particularly dexmedetomidine hydrochloride.

14. An oromucosal gel formulation for use according to any of claims 9 to 13, wherein dexmedetomidine or a pharmaceutically acceptable salt thereof, particularly dexmedetomidine hydrochloride, is administered in an amount of 50 to 200 µg/m², preferably from 70 µg/m² to 180 µg/m², more preferably from 100 µg/m² to 150 µg/m².

15. An oromucosal gel formulation for use according to any of claims 9 to 14, wherein the treatment is continued until the symptoms of separation anxiety gradually disappear or are reduced over time such that the animal no longer requires the treatment.

## Patentansprüche

1. Verbindung, die Dexmedetomidin, Medetomidin oder ein pharmazeutisch unbedenkliches Salz als der einzige Wirkstoff ist, für die Verwendung beim Behandeln von Trennungsangst bei Haus- und Nutztieren, insbesondere Hunden.

2. Verbindung für die Verwendung nach Anspruch 1, wobei Dexmedetomidin, Medetomidin oder ein pharmazeutisches Salz davon oromukosal verabreicht wird.

3. Verbindung für die Verwendung nach Anspruch 1 oder 2, wobei Dexmedetomidin, Medetomidin oder ein pharmazeutisches Salz davon in der Form eines halbfesten oromukosalem Gel verabreicht wird.

4. Verbindung für die Verwendung nach Anspruch 3, wobei das halbfeste oromukosale Gel in Bezug auf das Gewicht der Zusammensetzung 0,001 - 0,2 % (Gew./Gew.) Dexmedetomidin, Medetomidin oder eines pharmazeutisch unbedenklichen Salzes davon; 1 - 40 % (Gew./Gew.) eines Geliermittels; 0,2 - 10 % (Gew./Gew.) eines transmukosalen Durchdringungsverstärkers; 5 - 50 % (Gew./Gew.) eines wassermischbaren organischen Co-Lösungsmittels; und 30 - 80 % Wasser umfasst.

5. Verbindung für die Verwendung nach einem der Ansprüche 1 bis 4, wobei der Plasma-Cₘₐₓ-Wert von Dexmedetomidin in dem Subjekttier von 0,05 bis 0,8 ng/ml, vorzugsweise von 0,15 bis 0,6 ng/ml, stärker bevorzugt von etwa 0,2 bis etwa 0,5 ng/ml liegt.

6. Verbindung nach einem der Ansprüche 1 bis 4, wobei der Plasma-Cₘₐₓ-Wert von Medetomidin in dem Subjekttier von etwa 0,1 bis etwa 1,4 ng/ml, vorzugsweise von etwa 0,3 bis etwa 1,2 ng/ml, stärker bevorzugt von etwa 0,4 bis etwa 1,0 ng/ml liegt.

7. Verbindung für die Verwendung nach einem der Ansprüche 2 bis 5, wobei Dexmedetomidin oder ein pharmazeutisch unbedenkliches Salz davon oromukosal in einer Menge von 10 µg/m² bis 200 µg/m², vorzugsweise von 20 µg/m² bis 180 µg/m², stärker bevorzugt von 30 µg/m² bis 150 µg/m² verabreicht wird.

8. Verbindung für die Verwendung nach einem der Ansprüche 2, 3, 4 oder 6, wobei Medetomidin oder ein pharmazeutisch unbedenkliches Salz davon oromukosal in einer Menge von 20 µg/m² bis 400 µg/m², vorzugsweise von 40 µg/m² bis 360 µg/m², stärker bevorzugt von 60 µg/m² bis 300 µg/m² verabreicht wird.

9. Oromukosale Gelformulierung, umfassend in Bezug auf das Gewicht der Zusammensetzung 0,001 bis etwa 0,2 % (Gew./Gew.) Dexmedetomidin, Medetomidin oder eines pharmazeutisch unbedenklichen Salzes davon, 0,3 - 40 % (Gew./Gew.) eines Geliermittels; 0,2 - 15 % (Gew./Gew.) eines transmukosalen Durchdringungsverstärkers; 5 - 50 % (Gew./Gew.) eines wassermischbaren organischen Co-Lösungsmittels; und 30 - 80 % Wasser für die Verwendung beim Behandeln von Trennungsangst in Haus- und Nutztieren, insbesondere Hunden, wobei Dexmedetomidin, Medetomidin oder ein pharmazeutisch unbedenkliches Salz davon als der einzige Wirkstoff verabreicht wird, wobei eine Einzeldosis der Gelformulierung verabreicht wird und wobei die Dosis wirksam ist, um Symptome der Trennungsangst innerhalb von 60 Minuten, vorzugsweise innerhalb von 45 Minuten, am stärksten bevorzugt innerhalb von 30 Minuten zu mildern.

10. Oromukosale Gelformulierung für die Verwendung nach Anspruch 9, wobei die Wirkung wenigstens 8 Stunden anhält.

11. Oromukosale Gelformulierung für die Verwendung nach Anspruch 9 oder 10, umfassend in Bezug auf das Gewicht der Zusammensetzung 0,005 bis etwa 0,1 % (Gew./Gew.) Dexmedetomidin, Medetomidin oder eines pharmazeutisch unbedenklichen Salzes davon, 1 - 30 % (Gew./Gew.) eines Geliermittels; 0,5 - 10 % (Gew./Gew.) eines transmukosalen Durchdringungsverstärkers; 5 - 50 % (Gew./Gew.) eines wassermischbaren organischen Co-Lösungsmittels; und 40 - 70 % Wasser.

12. Oromukosale Gelformulierung für die Verwendung nach Anspruch 11, umfassend in Bezug auf das Gewicht der Zusammensetzung 0,005 bis etwa 0,05 % (Gew./Gew.) Dexmedetomidin, Medetomidin oder eines pharmazeutisch unbedenklichen Salzes davon, 10 - 25 % (Gew./Gew.) Hydroxypropylcellulose; 0,1 - 5 % (Gew./Gew.) Natriumlaurylsulphat; 15 - 40 % (Gew./Gew.) eines wassermischbaren organischen Co-Lösungsmittels; und 40 - 70 % Wasser.

13. Oromukosale Gelformulierung für die Verwendung nach Anspruch 12, umfassend in Bezug auf das Gewicht der Zusammensetzung 0,005 bis etwa 0,05 % (Gew./Gew.) Dexmedetomidin oder eines pharmazeutisch unbedenklichen Salzes davon, insbesondere Dexmedetomidinhydrochlorid.

14. Oromukosale Gelformulierung für die Verwendung nach einem der Ansprüche 9 bis 13, wobei Dexmedetomidin oder ein pharmazeutisches Salz davon, insbesondere Dexmedetomidinhydrochlorid in einer Menge von 50 bis 200 µg/m², vorzugsweise von 70 µg/m² bis 180 µg/m², stärker bevorzugt von 100 µg/m² bis 150 µg/m² verabreicht wird.

15. Oromukosale Gelformulierung für die Verwendung nach einem der Ansprüche 9 bis 14, wobei die Behandlung fortgesetzt wird, bis die Symptome der Trennungsangst allmählich verschwinden oder im Verlauf der Zeit derart reduziert werden, dass das Tier eine Behandlung nicht länger benötigt.

## Revendications

1. Composé qui est de la dexmédétomidine, de la médétomidine, ou un sel pharmaceutiquement acceptable de celle-ci, en tant que seul ingrédient actif pour l'utilisation dans le traitement d'anxiété de séparation chez des animaux de compagnie, particulièrement des chiens.

2. Composé pour l'utilisation selon la revendication 1, dans lequel la dexmédétomidine, la médétomidine, ou un sel pharmaceutiquement acceptable de celle-ci, est administré par voie oromuqueuse.

3. Composé pour l'utilisation selon la revendication 1 ou 2, dans lequel la dexmédétomidine, la médétomidine, ou un sel pharmaceutiquement acceptable de celle-ci, est administré sous la forme d'un gel oromuqueux semi-solide.

4. Composé pour l'utilisation selon la revendication 3, dans lequel le gel oromuqueux semi-solide comprend, en poids de la composition, 0,001 - 0,2 % (m/m) de dexmédétomidine, de médétomidine, ou d'un sel pharmaceutiquement acceptable de celle-ci ; 1 - 40 % (m/m) d'un agent gélifiant ; 0,2 - 10 % (m/m) d'un amplificateur de pénétration transmuqueuse ; 5 - 50 % (m/m) d'un co-solvant organique hydromiscible ; et 30 - 80 % (m/m) d'eau.

5. Composé pour l'utilisation selon l'une quelconque des revendications 1 à 4, dans lequel la valeur Cₘₐₓ plasmatique de dexmédétomidine chez l'animal sujet est de 0,05 à 0,8 ng/ml, de préférence de 0,15 à 0,6 ng/ml, de façon davantage préférée d'environ 0,2 à environ 0,5 ng/ml.

6. Composé pour l'utilisation selon l'une quelconque des revendications 1 à 4, dans lequel la valeur Cₘₐₓ plasmatique de médétomidine chez l'animal sujet est d'environ 0,1 à environ 1,4 ng/ml, de préférence d'environ 0,3 à environ 1,2 ng/ml, de façon davantage préférée d'environ 0,4 à environ 1,0 ng/ml.

7. Composé pour l'utilisation selon l'une quelconque des revendications 2 à 5, dans lequel la dexmédétomidine, ou un sel pharmaceutiquement acceptable de celle-ci, est administré par voie oromuqueuse en une quantité de 10 µg/m² à 200 µg/m², de préférence de 20 µg/m² à 180 µg/m², de façon davantage préférée de 30 µg/m² à 150 µg/m².

8. Composé pour l'utilisation selon l'une quelconque des revendications 2, 3, 4 ou 6, dans lequel la médétomidine, ou un sel pharmaceutiquement acceptable de celle-ci, est administré par voie oromuqueuse en une quantité de 20 µg/m² à 400 µg/m², de préférence de 40 µg/m² à 360 µg/m², de façon davantage préférée de 60 µg/m² à 300 µg/m².

9. Formule de gel oromuqueux comprenant, en poids de la composition, 0,001 à environ 0,2 % (m/m) de dexmédétomidine, de médétomidine, ou d'un sel pharmaceutiquement acceptable de celle-ci, 0,3 - 40 % (m/m) d'un agent gélifiant; 0,2 - 15 % (w /w) d'un amplificateur de pénétration transmuqueuse ; 5 - 50 % (w /w) d'un co-solvant organique hydromiscible ; et 30 - 80% (m/m) d'eau, pour l'utilisation dans le traitement d'anxiété de séparation chez des animaux de compagnie, particulièrement des chiens, dans laquelle la dexmédétomidine, la médétomidine, ou un sel pharmaceutiquement acceptable de celle-ci, est administré en tant que seul ingrédient actif, dans laquelle une seule dose de la formule de gel est administrée et dans laquelle la dose est efficace pour réduire des symptômes d'anxiété de séparation au sein d'une période de 60 minutes, de préférence au sein d'une période de 45 minutes, de façon préférée entre toutes au sein d'une période de 30 minutes.

10. Formule de gel oromuqueux pour l'utilisation selon la revendication 9, dans laquelle l'effet dure pendant au moins 8 heures.

11. Formule de gel oromuqueux pour l'utilisation selon la revendication 9 ou 10, comprenant, en poids de la composition, 0,005 à environ 0,1 % (m/m) de dexmédétomidine, de médétomidine, ou d'un sel pharmaceutiquement acceptable de celle-ci, 1 - 30 % (m/m) d'un agent gélifiant ; 0,5 - 10 % (m/m) d'un amplificateur de pénétration transmuqueuse ; 5 - 50 % (m/m) d'un co-solvant organique hydromiscible ; et 40 - 70 % (m/m) d'eau.

12. Formule de gel oromuqueux pour l'utilisation selon la revendication 11, comprenant, en poids de la composition, de 0,005 à environ 0,05 % (m/m) de dexmédétomidine, de médétomidine, ou d'un sel pharmaceutiquement acceptable de celle-ci, 10 - 25 % (m/m) d'hydroxypropylcellulose ; 0,1 - 5 % (m/m) de laurylsulfate de sodium ; 15 - 40 % (m/m) d'un co-solvant organique hydromiscible ; et 40 - 70 % (m/m) d'eau.

13. Formule de gel oromuqueux pour l'utilisation selon la revendication 12, comprenant, en poids de la composition, de 0,005 à environ 0,05 % (m/m) de dexmédétomidine ou d'un sel pharmaceutiquement acceptable de celle-ci, particulièrement de chlorhydrate de dexmédétomidine.

14. Formule de gel oromuqueux pour l'utilisation selon l'une quelconque des revendications 9 à 13,
dans laquelle la dexmédétomidine, ou un sel pharmaceutiquement acceptable de celle-ci, particulièrement du chlorhydrate de dexmédétomidine, est administré en une quantité de 50 à 200 µg/m², de préférence de 70 µg/m² à 180 µg/m², de façon davantage préférée de 100 µg/m² à 150 µg/m².

15. Formule de gel oromuqueux pour l'utilisation selon l'une quelconque des revendications 9 à 14,
dans laquelle le traitement est continué jusqu'à ce que les symptômes d'anxiété de séparation disparaissent progressivement ou soient réduits au fil du temps de telle sorte que l'animal ne nécessite plus le traitement.
